(19) Europäisches Patentamt
European Patent Office
Office européen des brevets

(11) Veröffentlichungsnummer: **0 103 540**
**A1**

(12) **EUROPÄISCHE PATENTANMELDUNG**

(21) Anmeldenummer: **83810357.0**

(22) Anmeldetag: **12.08.83**

(51) Int. Cl.³: **G 03 C 7/26**
// C07D211/46, C07D211/58

(30) Priorität: **18.08.82 CH 4938/82**

(43) Veröffentlichungstag der Anmeldung: **21.03.84**
**Patentblatt 84/12**

(84) Benannte Vertragsstaaten: **BE DE FR GB IT**

(71) Anmelder: **CIBA-GEIGY AG, Postfach, CH-4002 Basel (CH)**

(72) Erfinder: **Leppard, David G., Dr., Route de Bourguillon 6, CH-1723 Marly (CH)**
Erfinder: **Rody, Jean, Dr., Rütiring 82, CH-4125 Riehen (CH)**

(54) **Farbphotographisches Aufzeichnungsmaterial.**

(57) Verbindungen der Formel I

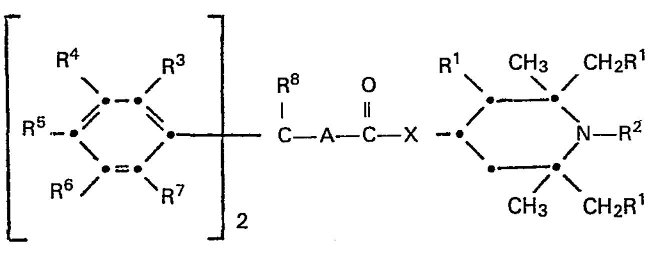

(I),

worin A eine direkte Bindung oder ein gegebenenfalls substituierter Alkylenrest ist, X —O— oder —N($R^{16}$)— ist, $R^2$ ein einwertiger Rest ist, $R^5$ oder $R^3$ Hydroxyl ist und $R^1$, $R^4$, $R^6$, $R^7$ und $R^8$ Wasserstoff oder einwertige Reste bedeuten, sind wirkungsvolle Stabilisatoren für photographische Farbstoffe und deren Vorstufen. Sie bewirken vor allem eine Erhöhung der Lichtbeständigkeit der entwickelten Farbbilder.

EP 0 103 540 A1

CIBA-GEIGY AG
Basel (Schweiz)

3-14052/-

Farbphotographisches Aufzeichnungsmaterial

Die vorliegende Erfindung betrifft ein farbphotographisches Aufzeichnungsmaterial, das in mindestens einer lichtempfindlichen Silberhalogenidemulsionsschicht und/oder in mindestens einer der üblichen Hilfsschichten mindestens ein spezifisches Polyalkylpiperidin-Lichtschutzmittel enthält.

Polyalkylpiperidine sind als sterisch gehinderte Amine allgemein als Lichtschutzmittel für organische Materialien, insbesondere für Polymere, bekannt. Es wurde auch bereits in der DE-OS 2 126 954 vorgeschlagen, solche Polyalkylpiperidine als Mittel gegen das Ausbleichen von Farbphotographien zu verwenden. Es wurde weiterhin in der EP-A 11051 vorgeschlagen, als Lichtschutzmittel für Farbphotographien bestimmte Polyalkylpiperidinderivate zu verwenden, die mindestens eine Phenolgruppe enthalten. Es handelt sich dabei um Polyalkylpiperidinester von Hydroxylbenzylmalonsäuren.

In Weiterverfolgung dieser Forschungsarbeiten wurde gefunden, dass Polyalkylpiperidinderivate von Diphenolalkancarbonsäuren ebenfalls eine ausgezeichnete Lichtschutzwirkung für Farbphotographien besitzen und darüber hinaus auch eine Stabilisierung der Farbstoffe gegenüber Veränderungen durch Einwirkung von Wärme oder Feuchtigkeit bewirken.

Gegenstand der vorliegenden Erfindung ist daher ein farbphotographisches Aufzeichnungsmaterial, das in mindestens einer lichtempfindlichen Silberhalogenidemulsionsschicht, einer Zwischenschicht und/oder einer Schutzschicht mindestens eine Polyalkylpiperidinverbindung als

Lichtschutzmittel enthält und das dadurch gekennzeichnet ist, dass es als Lichtschutzmittel eine Verbindung der Formel I enthält

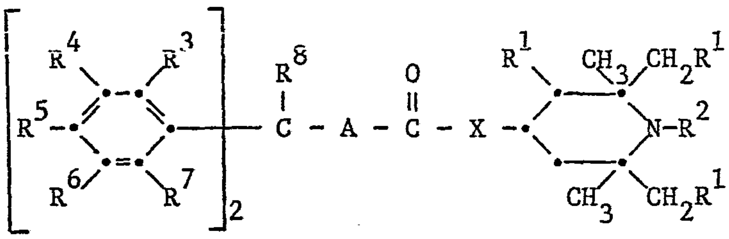

(I),

worin

$R^1$ Wasserstoff oder Methyl ist,

$R^2$ Hydroxyl, $C_1$-$C_{12}$-Alkyl, $C_3$-$C_6$-Alkenyl, $C_3$-$C_4$-Alkinyl, $C_7$-$C_{12}$-Aralkyl, Glycidyl, durch Halogen, Cyan, $-COOR^9$ oder $-CON(R^{10})(R^{11})$ substituiertes $C_1$-$C_4$-Alkyl, eine Gruppe $-CO-R^{12}$, $-CO-OR^9$ oder $-CO-N(R^{10})(R^{11})$, eine Gruppe $-CH_2-CH(R^{13})-OR^{14}$, $-SO-R^{15}$, $-SO_2-R^{15}$, $-OR^9$, $-OOC-R^{12}$ oder eine Gruppe der Formel

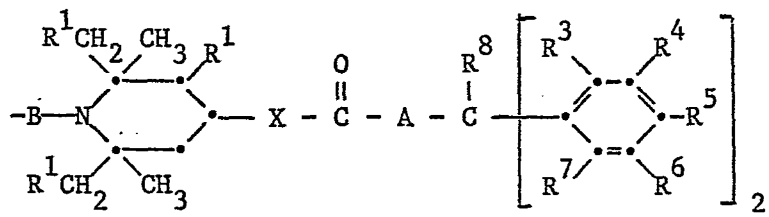

bedeutet,

$R^3$ und $R^5$ Wasserstoff oder Hydroxyl sind, wobei entweder $R^3$ Wasserstoff und $R^5$ Hydroxyl oder $R^3$ Hydroxyl und $R^5$ Wasserstoff sind,

$R^4$ $C_1$-$C_{12}$-Alkyl, $C_5$-$C_6$-Cycloalkyl, $C_6$-$C_{10}$-Aryl oder $C_7$-$C_9$-Phenylalkyl ist,

$R^6$ und $R^7$ unabhängig voneinander Wasserstoff, $C_1$-$C_{12}$-Alkyl, $C_5$-$C_8$-Cycloalkyl, $C_6$-$C_{10}$-Aryl oder $C_7$-$C_9$-Phenylalkyl bedeuten,

$R^8$ Wasserstoff, $C_1$-$C_{12}$-Alkyl, $C_5$-$C_8$-Cycloalkyl, Benzyl oder Phenyl ist,

$R^9$ $C_1$-$C_{12}$-Alkyl, Allyl, Cyclohexyl oder Benzyl bedeutet,

$R^{10}$ $C_1$-$C_{12}$-Alkyl, Allyl, Cyclohexyl, Benzyl, Phenyl oder $C_7$-$C_{12}$-Alkylphenyl bedeutet,

$R^{11}$ Wasserstoff, $C_1$-$C_{12}$-Alkyl, Allyl, Cyclohexyl oder Benzyl bedeutet oder

$R^{10}$ und $R^{11}$ zusammen mit dem N-Atom, an das sie gebunden sind, einen 5- oder 6-gliedrigen heterocyclischen Ring bilden,

$R^{12}$ Wasserstoff, $C_1$-$C_{12}$-Alkyl, $C_2$-$C_6$-Alkenyl, Chlormethyl, $C_5$-$C_8$-Cycloalkyl, $C_7$-$C_{12}$-Aralkyl, Phenyl, $C_7$-$C_{12}$-Alkylphenyl oder durch 1 oder 2 $C_1$-$C_4$-Alkylgruppen und eine Hydroxygruppe substituiertes Phenyl, Phenylmethyl oder Phenylethyl bedeutet,

$R^{13}$ Wasserstoff, $C_1$-$C_4$-Alkyl, $C_2$-$C_{13}$-Alkoxymethyl, Phenyl oder Phenoxymethyl bedeutet,

$R^{14}$ Wasserstoff, $C_1$-$C_{12}$-Alkyl, eine Gruppe $-CO-R^{12}$, $-CO-N(R^{10})(R^{11})$ oder eine Gruppe der Formel II bedeutet

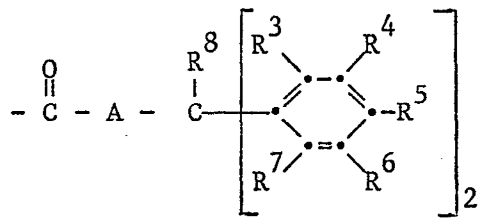 (II),

$R^{15}$ $C_1$-$C_{12}$-Alkyl, Phenyl oder $C_7$-$C_{12}$-Alkylphenyl ist,

A eine direkte Bindung oder $C_1$-$C_{12}$-Alkylen, das durch -S- oder -COOunterbrochen oder durch eine der Gruppen $-SO_2-R^{15}$, -CN, $-P(O)(OR^{15})_2$ oder

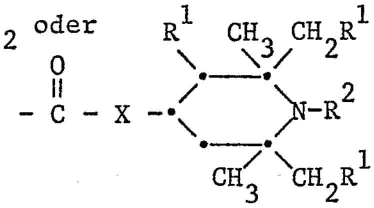

substituiert sein kann, bedeutet,

B $C_2$-$C_{12}$-Alkylen, $C_4$-$C_8$-Alkenylen, $C_4$-$C_6$-Alkinylen oder $C_8$-$C_{14}$-Aralkylen bedeutet, und

X -O- oder $-N(R^{16})-$ bedeutet, worin

$R^{16}$ Wasserstoff, $C_1$-$C_{18}$-Alkyl, $C_5$-$C_{12}$-Cycloalkyl oder eine Gruppe der Formel III

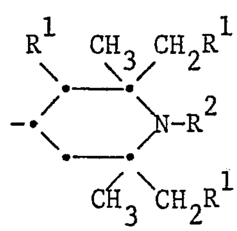 (III)

bedeutet.

Darin können die Substituenten $R^2$, $R^4$, $R^6$, $R^7$, $R^8$, $R^9$,$R^{10}$,$R^{11}$,$R^{12}$,$R^{14}$, und $R^{15}$ $C_1$-$C_{12}$-Alkyl sein und sie können als solches verzweigtes oder unverzweigtes Alkyl sein wie z.B. Methyl, Ethyl, Isopropyl, tert.Butyl, Isoamyl, n-Hexyl, n-Octyl, 2-Ethylhexyl, 1,1,3,3-Tetramethylbutyl, Isononyl, n-Decyl oder n-Dodecyl. $R^{16}$ als $C_1$-$C_{18}$-Alkyl kann darüber hinaus auch z.B. Tetradecyl, Hexadecyl oder Octadecyl sein.

$R^2$ als Alkenyl können z.B. Allyl, Methallyl, Dimethylallyl oder 2-Hexenyl sein. $R^{12}$ als Alkenyl kann darüber hinaus auch Vinyl sein. $R^2$ als Alkinyl kann z.B. 2-Propinyl oder 2-Butinyl sein.

$R^4$, $R^6$, $R^7$, $R^8$ und $R^{12}$ als Cycloalkyl können z.B. Cyclopentyl, Cyclohexyl, Methylcyclohexyl, Dimethylcyclohexyl, Cycloheptyl oder Cyclooctyl sein. $R^{16}$ als $C_5$-$C_{12}$-Cycloalkyl kann darüber hinaus auch z.B. Diethylcyclohexyl oder Cyclododecyl sein.

$R^4$, $R^6$ und $R^7$ als Phenylalkyl können z.B. Benzyl, Phenylethyl oder Phenylpropyl sein. $R^2$, $R^{12}$ als Aralkyl können darüber hinaus auch Phenylbutyl oder Naphthylmethyl sein.

$R^4$, $R^6$ und $R^7$ als Aryl können z.B. Phenyl oder Naphthyl sein, $R^{10}$, $R^{12}$, $R^{15}$ als Alkylphenyl kann z.B. Tolyl, Xylyl, Isopropylphenyl, tert.Butylphenyl oder Diethylphenyl sein.

Das Zwischenglied A als $C_1$-$C_{12}$-Alkylen kann unverzweigtes oder verzweigtes Alkylen sein, wie z.B. Methylen, 1,2-Ethylen, 1,2-Propylen, 1,3-Propylen, 1,2-Butylen, 1,4-Butylen, Hexamethylen, Octamethylen oder Dodecamethylen. Bevorzugt ist A als Alkylen $C_1$-$C_6$-Alkylen, insbesondere $C_1$-$C_4$-Alkylen.

Das Bindeglied B kann $C_2$-$C_{12}$-Alkylen sein und ist als solches vorzugsweise unverzweigtes Alkylen, wie z.B. 1,2-Ethylen, 1,3-Propylen,

Tetra-, Hexa-, Octa- oder Dodecamethylen. B kann auch Alkenylen sein, wie z.B. 2-Butenylen-1,4 oder 3-Hexenylen-1,6. B kann auch Alkinylen sein, wie z.B. 2-Butinylen-1,4 oder 3-Hexinylen-1,6. B kann auch Aralkylen sein, wie z.B. Xylylen oder Bitolylen.
$R^{10}$ und $R^{11}$ können zusammen mit dem N-Atom, an das sie gebunden sind, einen 5- oder 6-gliedrigen heterocyclischen Ring bilden. Dies kann z.B ein Pyrrolidin-, Piperidin-, Morpholin- oder 4-Alkylpiperazinring sein.

Bevorzugt sind als Lichtschutzmittel Verbindungen der Formel I, worin $R^1$ Wasserstoff ist, $R^2$ Hydroxy, $C_1$-$C_4$-Alkoxy, Acetoxy, $C_1$-$C_4$-Alkyl, $C_3$-$C_4$-Alkenyl, Propargyl, Glycidyl, Benzyl, durch $-COOR^9$ substituiertes Methyl oder Ethyl, eine Gruppe $-CO-R^{12}$, $-CO-OR^9$ oder $-CO-N(R^{10})(R^{11})$, eine Gruppe $-CH_2-CH(R^{13})-OR^{14}$, $-SO-R^{15}$, $-SO_2-R^{15}$, $-OR^9$ oder $-OOC-R^{12}$ bedeutet, $R^3$ Wasserstoff und $R^5$ Hydroxyl oder $R^3$ Hydroxyl und $R^5$ Wasserstoff sind, $R^4$ $C_1$-$C_8$-Alkyl ist, $R^6$ und $R^7$ unabhängig voneinander H oder $C_1$-$C_8$-Alkyl sind, $R^8$ Wasserstoff oder $C_1$-$C_{12}$-Alkyl ist, $R^9$ $C_1$-$C_8$-Alkyl, Allyl oder Cyclohexyl ist, $R^{10}$ $C_1$-$C_{12}$-Alkyl, Cyclohexyl oder Phenyl ist, $R^{11}$ Wasserstoff oder $C_1$-$C_{12}$-Alkyl ist oder $R^{10}$ und $R^{11}$ zusammen mit dem N-Atom, an das sie gebunden sind, einen 6-gliedrigen heterocyclischen Ring bilden, $R^{12}$ $C_1$-$C_{12}$-Alkyl, $C_2$-$C_4$-Alkenyl, Cyclohexyl, Benzyl, Phenyl oder 2-(3,5-Di-tert.butyl-4-hydroxyl)-ethyl ist, $R^{13}$ Wasserstoff, Methyl oder Phenyl ist, $R^{14}$ Wasserstoff, $C_1$-$C_4$-Alkyl, eine Gruppe $-CO-R^{12}$, $-CO-N(R^{10})(R^{11})$ oder eine Gruppe der Formel II ist, $R^{15}$ $C_1$-$C_4$-Alkyl, Phenyl oder Tolyl ist, A eine direkte Bindung oder $C_1$-$C_6$-Alkylen, das durch -CN oder eine Gruppe der Formel IV

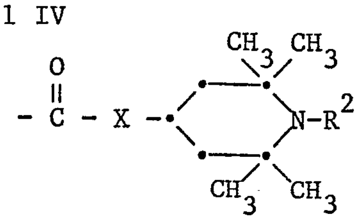

(IV)

substituiert sein kann und X -O- oder -NH- bedeutet.

Bevorzugt sind als Lichtschutzmittel insbesondere Verbindungen der Formel I, worin $R^1$ Wasserstoff ist, $R^2$ Methoxy, Methyl, Allyl, Benzyl, Acetyl, Acryloyl, eine Gruppe $-CO-N(R^{10})(R^{11})$, $-SO-R^{15}$ oder $-SO_2-R^{15}$ ist, $R^3$ Wasserstoff ist, $R^4$ $C_1-C_6$-Alkyl, $R^5$ Hydroxyl, $R^6$ und $R^7$ unabhängig voneinander Wasserstoff oder $C_1-C_4$-Alkyl sind, $R^8$ Wasserstoff oder $C_1-C_6$-Alkyl ist, $R^{10}$ $C_1-C_8$-Alkyl, Cyclohexyl oder Phenyl und $R^{11}$ Wasserstoff oder $C_1-C_8$-Alkyl ist, $R^{15}$ Methyl, Phenyl oder p-Tolyl ist, A eine direkte Bindung oder $C_1-C_4$-Alkylen ist, das durch eine Gruppe der Formel IV substituiert sein kann und X -O- oder -NH- bedeutet.

Besonders bevorzugt als Lichtschutzmittel sind die Verbindungen der Formel I, worin $R^1$ Wasserstoff ist, $R^2$ Methoxy, Methyl, Allyl, Benzyl, Acetyl, Acryloyl oder eine Gruppe $-CO-N(R^{10})(R^{11})$, $-SO-R^{15}$ oder $-SO_2R^{15}$ ist, $R^3$ Wasserstoff, $R^4$ $C_1-C_4$-Alkyl und $R^5$ Hydroxyl sind, $R^6$ Wasserstoff oder $C_1-C_4$-Alkyl ist, $R^7$ und $R^8$ Wasserstoff oder Methyl sind, $R^{10}$ $C_1-C_4$-Alkyl, Cyclohexyl oder Phenyl ist, $R^{11}$ Wasserstoff oder $C_1-C_4$-Alkyl ist, $R^{15}$ Phenyl oder p-Tolyl ist, A eine direkte Bindung oder $C_1-C_4$-Alkylen ist und X -O- ist, insbesondere solche Verbindungen der Formel I worin $R^1$ Wasserstoff ist, $R^2$ Methyl, Allyl, Benzyl, Acetyl oder Acryloyl ist, $R^3$ Wasserstoff, $R^4$ $C_1-C_4$-Alkyl, $R^5$ Hydroxyl und $R^6$, $R^7$ und $R^8$ Wasserstoff oder Methyl sind, A $C_1-C_4$-Alkylen ist und X -O- ist.

Ein Teil der hier beschriebenen Verbindungen der Formel I ist bekannt aus der DE-OS 27 17 087, wo die Verbindungen als Lichtschutzmittel für Kunststoffe beschrieben sind. Dort sind auch verschiedene Verfahren zur Herstellung dieser Verbindungen beschrieben. Diese umfassen erstens die Umsetzung von Diphenylalkancarbonsäuren oder deren Estern oder Halogeniden mit 4-Hydroxy- oder 4-Aminopolyalkylpiperidinen und zweitens die Reaktion von aliphatischen Ketocarbonsäureestern oder -amiden von 4-Hydroxy- oder 4-Aminopolyalkylpiperidinen mit Phenolen gemäss dem Verfahren der DE-OS 1 953 333.

Die Einführung des Substituenten $R^2$ kann in verschiedenen Stufen der Herstellung durch Substitution der entsprechenden NH-Verbindungen geschehen nach der allgemein für die N-Substitution sekundärer Amine bekannten Methoden.

Soweit die hier beschriebenen Verbindungen der Formel I nicht zu den in der DE-OS 27 17 087 beschriebenen Verbindungen gehören, können sie analog dazu hergestellt werden. Nähere Details hierzu können den später folgenden Herstellungsbeispielen entnommen werden.

Beispiele einzelner Verbindungen der Formel I, wie sie gemäss der Erfindung als Lichtschutzmittel für farbphotographisches Aufzeichnungsmaterial verwendet werden können, sind die folgenden Verbindungen:

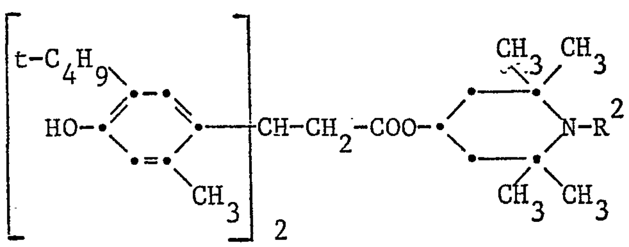

Nr. 1 $R^2 = -CH_3$

Nr. 2 $R^2 = -CO-CH_3$

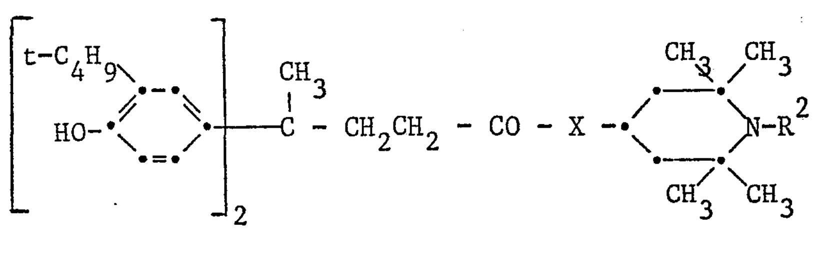

Nr. 3 X = -O-

$R^2$ = 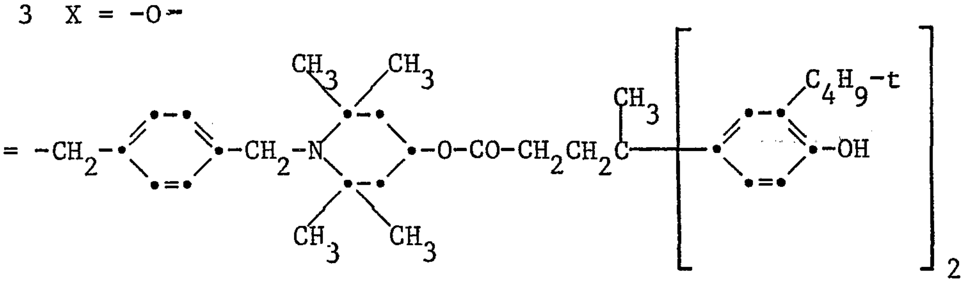

Nr. 4 X = -O- $R^2$ = $-CH_2CH=CH_2$

Nr. 5 X = -NH- $R^2$ = $-CO-CH=CH_2$

Nr. 6 X = -O- $R^2$ = $-CO-CH=CH_2$

Nr. 7 X = -O- $R^2$ = 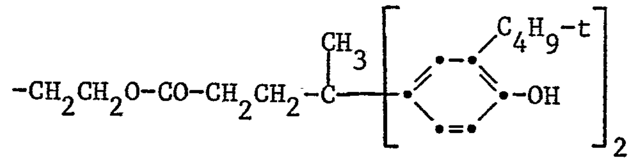

Nr. 8 X = -O- $R^2$ = $-CH_3$

Nr. 8a X = $-N(C_4H_9)-$ $R^2$ = $-CH_3$

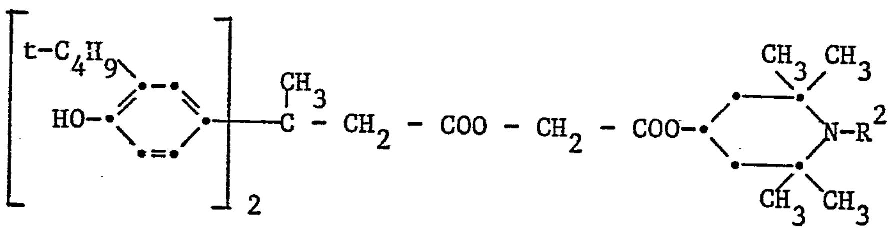

Nr. 9 $R^2$ = $-CH_3$

Nr. 10 $R^2$ = $-SO_2-CH_3$

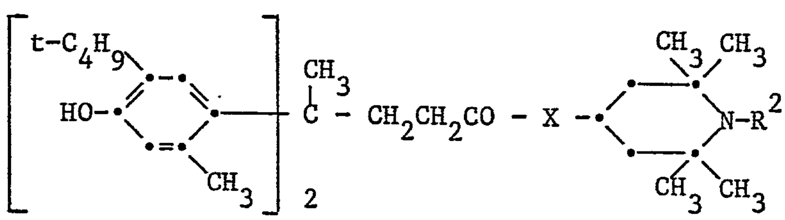

Nr. 11 X = -O- $R^2$ = $-CO-CH=CH_2$

Nr. 12 X = -O- $R^2$ = $-CO-N(CH_3)_2$

Nr. 13 X = -O- $R^2$ = $-CH_2CH_2OH$

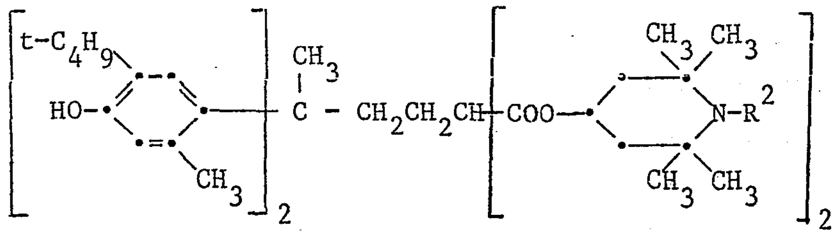

Nr. 14 $R_2$ = $-CO-CH=CH_2$

Nr. 15 $R_2$ = $-CH_3$

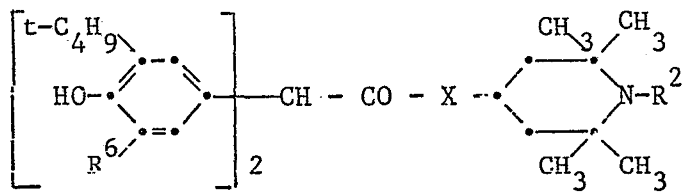

Nr. 16 X = -O- $R^2$ = $CH_3$ $R^6$ = $t-C_4H_9$

Nr. 17 X = -O- $R^2$ = Benzyl $R^6$ = $t-C_4H_9$

Nr. 18 X = -O- $R^2$ = $-CO-CH_3$ $R^6$ = $t-C_4H_9$

Nr. 19 X = -O- $R^2$ = $-CO-CH_3$ $R^6$ = $CH_3$

Nr. 20 X = -O- $R^2$ = $-CO-CH=CH_2$ $R^6$ = $t-C_4H_9$

Nr. 21 X = -O- $R^2$ = $-OCH_3$ $R^6$ = $t-C_4H_9$

Nr. 22 X = -O- $R^2$ = $-CO-N(C_2H_5)_2$ $R^6$ = $CH_3$

Nr. 23 X = -O- $R^2$ = $-CH_2CH_2-OOC-CH-$ 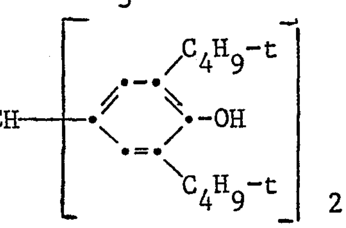 $R^6$ = $t-C_4H_9$

Die Verbindungen der Formel I sind in Wasser kaum löslich und sie werden deshalb den photographischen Schichten vorzugsweise als Dispersion oder Emulsion zugesetzt. In der Regel werden die Stabilisatoren zusammen mit den Farbkupplern in das photographische Material eingearbeitet.

Hierzu löst man die Verbindungen der Formel I zusammen mit den Farbkupplern und gegebenenfalls mit weiteren Lichtschutzmitteln in einem niedrigsiedenden organischen Lösungsmittel, wie Methylacetat, Ethylacetat, Tetrachlorkohlenstoff, Chloroform, Methanol, Ethanol, n-Butanol, Dioxan, Aceton oder Benzol, einem hochsiedenden organischen Lösungsmittel, wie Tricresylphosphat, Dimethylformamid, Dimethylsulfoxid, Din-butylphthalat oder Ethyl-N-diphenylcarbamat, oder einem Lösungsmittelgemisch aus den oben erwähnten niedrigsiedenden und hochsiedenden organischen Lösungsmitteln, gibt die erhaltene Lösung zu einer Schutzkolloidlösung, wie insbesondere einer wässrigen Gelatinelösung, zu und dispergiert darin die Lösung mittels einer Kolloidmühle, eines Homogenisators oder durch Anwendung von Ultraschall.

Die so erhaltenen Dispersionen werden dann zur Herstellung der Schichten von farbphotographischen Aufzeichnungsmaterialien verwendet. Diese Schichten können z.B. Zwischen- oder Schutzschichten, insbesondere jedoch lichtempfindliche (blau-, grün- und rotempfindliche) Silberhalogenidemulsionsschichten sein, in denen bei der Entwicklung des belichteten Aufzeichnungsmaterials aus den entsprechenden Farbkupplern, die Blaugrün (Cyan)-, Purpur (Magenta)- und Gelbfarbstoffe gebildet werden.

Gegebenenfalls kann das Lichtschutzmittel auch in den Behandlungsbädern appliziert werden, die nach der Farbentwicklung verwendet werden, z.B. in Fixier- und/oder Waschbädern, wobei jedoch eine gewisse Löslichkeit der Verbindungen der Formel I in Alkoholen (Methanol/Ethanol), wässrigem Alkali oder Wasser erforderlich ist. Wenn die Diffusionstransfermethode angewandt wird, kann das Lichtschutzmittel auch in eine Empfangsschicht eingearbeitet werden.

Die Silberhalogenidschichten können beliebige Farbkuppler, insbesondere Blaugrün-, Purpur- und Gelb-Kuppler, die zur Bildung der genannten Farbstoffe und damit der Farbbilder verwendet werden, enthalten.

Im photographischen Aufzeichnungsmaterial gemäss vorliegender Erfindung können die Lichtschutzmittel der Formel I ausser mit den Farbkupplern zusätzlich auch mit Ultraviolettabsorbern oder anderen Lichtschutzmitteln in der gleichen Schicht kombiniert werden.

Die Silberhalogenidemulsionen enthalten als Bindemittel vorzugsweise Gelatine, gegebenenfalls im Gemisch mit anderen hochmolekularen natürlichen oder synthetischen Verbindungen.

Die Silberhalogenidemulsionen können beispielsweise Silberbromid-, Silberchlorid- oder Silberjodidemulsionen oder auch solche Emulsionen sein, die ein Gemisch von Silberhalogeniden enthalten, wie z.B. Silberbromid-jodid- oder Silberchlorid-bromid-emulsionen.

Die Emulsionen können chemisch sensibilisiert werden, sie können ferner übliche organische Stabilisatoren und Antischleiermittel sowie auch übliche Weichmacher, wie z.B. Glycerin, enthalten. Die Emulsionen können ferner mit den für Gelatine üblichen Härtungsmitteln gehärtet werden. Ferner können die Emulsionen übliche Giesshilfsmittel enthalten. Die Emulsionen können auf übliche Schichtträger für photographisches Aufzeichnungsmaterial aufgebracht werden.

Zur Entwicklung des farbphotographischen Aufzeichnungsmaterials können die üblichen Entwicklerbäder eingesetzt werden. Diese enthalten in der Regel eine Entwicklersubstanz des p-Phenylendiamin-Typs, einen Entwicklungsverzögerer, wie Kaliumbromid, ein Antioxydationsmittel, wie Natriumsulfit oder Hydroxylamin, und eine Base, z.B. ein Alkalihydroxid oder Alkalicarbonat. Ferner können die Entwicklungsbäder übliche Antischleiermittel und Komplexbildner enthalten.

Die erfindungsgemäss einzusetzenden Lichtschutzmittel eignen sich in gewissen Fällen auch zum Schutz farbphotographischer Schichten, in denen die Farbstoffe direkt in die Emulsion eingelagert werden und das Bild durch selektive Bleichung erzeugt wird.

Die Menge des Lichtschutzmittels oder der Lichtschutzmittel kann in weiten Grenzen schwanken und liegt etwa im Bereich von 1 bis 2000 mg, vorzugsweise 100 bis 800 und insbesondere 200 - 500 mg pro $m^2$ der Schicht, in die es (sie) eingearbeitet wird (werden).

Falls das photographische Material ein UV-Strahlung absorbierendes Mittel enthält, so kann dieses mit dem Lichtschutzmittel zusammen in einer Schicht oder auch in einer benachbarten Schicht vorhanden sein. Das Gewichtsverhältnis zwischen dem Ultraviolettabsorber und dem Lichtschutzmittel der Formel I beträgt etwa (2-10):1, das molare Verhältnis etwa (5-20):1. Beispiele für Ultraviolett-Absorber sind Verbindungen vom Benzophenon-, Acrylnitril-, Thiazolidon-, Benztriazol-, Oxazol-, Thiazol- und Imidazoltyp.

Die mit dem erfindungsgemässen Aufzeichnungsmaterial durch Belichtung und Entwicklung erhaltenen Farbbilder zeigen eine sehr gute Lichtechtheit gegenüber sichtbarem und ultraviolettem Licht. Die Verbindungen der Formel I sind praktisch farblos, so dass es zu keiner Verfärbung der Bilder kommt; ausserdem sind sie gut verträglich mit den üblichen, in den einzelnen Schichten vorhandenen photographischen Zusatzstoffen. Aufgrund ihrer guten Wirksamkeit kann man ihre Einsatzmenge herabsetzen und vermeidet so ihre Ausfällung oder ihr Auskristallisieren, wenn man sie als organische Lösung in die wässerigen Bindemittelemulsionen, die für die Herstellung photographischer Schichten verwendet werden, einarbeitet. Die einzelnen nach der Belichtung des photographischen Aufzeichnungsmaterials notwendigen Verarbeitungsschritte zur Herstellung der Farbbilder werden durch die Lichtschutzmittel nicht nachteilig beeinflusst. Ferner kann die bei blauempfindlichen Emulsionen häufig auftretende sogenannte Druckschleierbildung weitgehend zurückgedrängt werden. Diese kann z.B. auftreten, wenn auf photographische Materialien (Silberhalogenidemulsionsschichten, die sich auf einem Träger aus natürlichen oder synthetischen Materialien befinden) mechanische Beanspruchungen, z.B. Drehen, Biegen oder Reiben, während

der Herstellung oder während der Behandlung vor der Entwicklung ausgeübt werden. (T.H. James, The Theory of Photographic Process 4. Auflage, Macmillan, New York, N.Y. 1977, Seite 23 ff., S. 166 ff.).

Die nachfolgenden Beispiele erläutern die vorliegende Erfindung, ohne sie einzuschränken. Die Temperatur ist darin in °C angegeben.

Beispiele der Herstellung der Lichtschutzmittel

Beispiel 1: 8,5 g (0,02 Mol) 4,4-Bis-(3-tert.-butyl-4-hydroxyphenyl)-valeriansäureethylester werden mit 3,1 g (0,02 Mol) 4-Hydroxy-2,2,6,6-tetramethylpiperidin in 200 ml Xylol gelöst. Nach der Zugabe von 0,1 g Dibutylzinnoxid wird ca. 6 Stunden unter Stickstoff zum Sieden erhitzt, wobei das sich bildende Ethanol fortlaufend abdestilliert wird. Wenn kein Ethanol mehr abdestilliert, wird die Reaktionslösung auf Raumtemperatur abgekühlt, mit 200 ml Wasser versetzt und gut durchgemischt. Die organische Phase wird getrennt, über Magnesiumsulfat getrocknet und eingedampft. Das entstehende Oel wird säulenchromatographisch gereinigt. So erhält man 4,4-Bis-(3-tert.-butyl-4-hydroxy-phenyl)-valeriansäure-(2,2,6,6-tetramethylpiperidin-4-yl)-ester als farblosen Feststoff mit einem Smp. von 100°.

Verwendet man anstelle von 4-Hydroxy-2,2,6,6-tetramethylpiperidin eine entsprechende Menge 1-Allyl-4-hydroxy-2,2,6,6-tetramethylpiperidin und verfährt man im übrigen wie oben beschrieben, so erhält man den 4,4-Bis-(3-tert.-butyl-4-hydroxyphenyl)-valeriansäure-(1-allyl-2,2,6,6-tetramethylpiperidin-4-yl)-ester mit Smp. 75° (Lichtschutzmittel Nr. 4).

Analog werden 4,4-Bis-(3-tert.-butyl-4-hydroxyphenyl)-valeriansäure-(1-methyl-2,2,6,6-tetramethylpiperidin-4-yl)-ester, Smp. 197° (Lichtschutzmittel Nr. 8) und die Lichtschutzmittel der folgenden Formeln hergestellt:

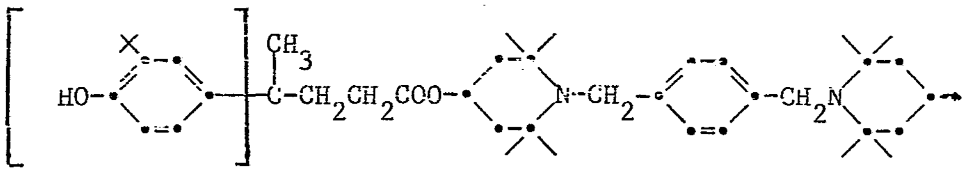

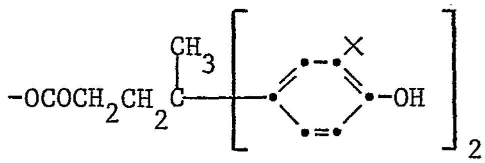

Lichtschutzmittel Nr. 3, Smp. 165°

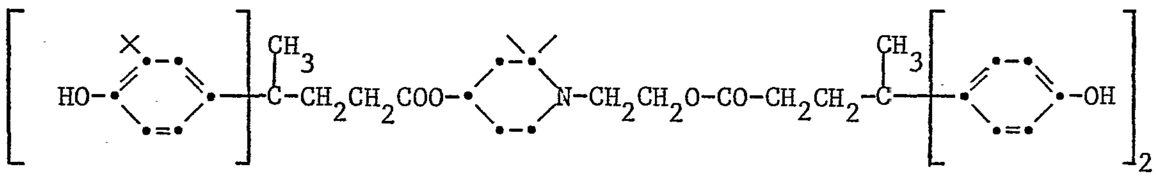

Lichtschutzmittel Nr. 7, Smp. 200°.

Analog erhält man aus Bis-(3,5-di-tert—butyl-4-hydroxyphenyl)-essigsäure-methylester und den entsprechenden 4-Hydroxypiperidinen den Bis-(3,5-di-tert-butyl-4-hydroxyphenyl)-essigsäure-(1,2,2,6,6-pentamethylpiperidin-4-yl)-ester mit Smp. 149° (Lichtschutzmittel Nr. 16) den -(1-benzyl-2,2,6,6-tetramethylpiperidin-4-yl)-ester mit Smp. 138° (Lichtschutzmittel Nr. 17) sowie den Diester der Formel

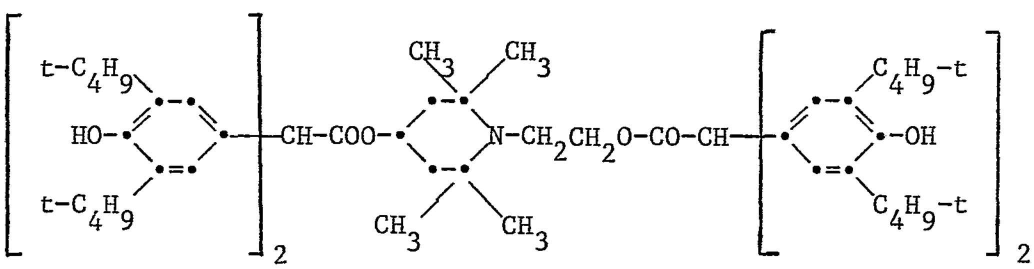

der bei 237° schmilzt (Stabilisator Nr. 23).

Analog erhält man aus 3,3-Bis(3-tert-butyl-4-hydroxybenzyl)-buttersäure-methylester den entsprechenden -(1,2,2,6,6-pentamethylpiperidin-4-yl)-ester mit Smp. 110° (Lichtschutzmittel Nr. 9).

Beispiel 2: Durch analoge Umsetzung von 4,4-Bis-(3-tert-butyl-4-hydroxyphenyl)-valeriansäure-ethylester mit 4-Amino-2,2,6,6-tetramethylpiperidin erhält man das 4-[4,4-Bis-(3-tert-butyl-4-hydroxyphenyl)-valeriansäureamido]-2,2,6,6-tetramethylpiperidin mit Smp. 133°.

2,7 g dieser Verbindung (0,005 Mol) werden in 80 ml Ethylacetat gelöst. Nach der Zugabe von 0,6 g (0,006 Mol) Triethylamin wird die Lösung auf -5° gekühlt und eine Lösung von 0,55 g (0,006 Mol) Acrylsäurechlorid in 10 ml Ethylacetat tropfenweise zugegeben. Das Gemisch wird weitere 3 Stunden bei -5° gerührt und auf Raumtemperatur erwärmt. Nach Zugabe von 5 ml Methanol wird die Lösung mit Wasser gewaschen, über Magnesiumsulfat getrocknet und eingedampft. Das entstehende Oel wird durch Säulenchromatographie gereinigt. Man erhält so das 4-[4,4-Bis-(3-tert-butyl-4-hydroxyphenyl)-valerianamido]-1-acroyl-2,2,6,6-tetramethylpiperidin mit Smp. 110° (Lichtschutzmittel Nr. 5).

Verwendet man anstelle von 4-[4,4-Bis-(3-tert-butyl-4-hydroxyphenyl)-valerianamido]-2,2,6,6-tetramethylpiperidin eine entsprechende Menge 4,4-Bis-(3-tert-butyl-4-hydroxyphenyl)-valeriansäure-(2,2,6,6-tetramethylpiperidin-4-yl)-ester und verfährt man im übrigen wie oben beschrieben, so erhält man 4,4-Bis-(3-tert-butyl-4-hydroxyphenyl)-valeriansäure-(1-acroyl-2,2,6,6-tetramethylpiperidin-4-yl)-ester mit Smp. 95° (Lichtschutzmittel Nr. 6).

Verwendet man anstelle von 4-[4,4-Bis-(3-tert-butyl-4-hydroxyphenyl)-valerianamido)-2,2,6,6-tetramethylpiperidin eine entsprechende Menge 3,3-Bis-(3-tert-butyl-6-methyl-4-hydroxyphenyl)-butyl-malonsäure-bis-(2,2,6,6-tetramethylpiperidin-4-yl)-ester und verfährt im übrigen wie oben beschrieben, so erhält man [3,3-Bis-(3-tert-butyl-6-methyl-4-hydroxyphenyl)-butyl]-malonsäure-bis-(1-acroyl-2,2,6,6-tetramethylpiperidin-4-yl)-ester als leicht gelbes Oel (Lichtschutzmittel Nr. 14).

Beispiel 3: 10 g 2,2,6,6-Tetramethyl-4-[3,3-bis-(3-tert-butyl-6-methyl-4-hydroxyphenyl)propionyloxy]-piperidin werden in 25 ml Acetanhydrid 6 Stunden bei 110° gerührt. Die gebildete Essigsäure und das überschüssige Anhydrid werden anschliessend unter Vakuum möglichst vollständig abdestilliert. Der Rückstand wird nach dem Abkühlen auf Raumtemperatur mit 100 ml Wasser versetzt, gut durchgemischt, abfiltriert, mit Wasser gewaschen und getrocknet. Nach Kristallisation aus Acetonitril erhält man das 1-Acetyl-2,2,6,6-tetramethyl-4-[3,3-bis-(3-tert-butyl-6-methyl-4-hydroxyphenyl)propionyloxy]-piperidin mit einem Smp. von 270° (Lichtschutzmittel Nr. 2).

In analoger Weise erhält man den Bis-(3,5-di-tert-butyl-4-hydroxyphenyl)-essigsäure-(1-acetyl-2,2,6,6-piperidin-4-yl)-ester mit Smp. 198° (Stabilisator Nr. 18).

Anwendungsbeispiele

Beispiel A

0,087 g des Gelbkupplers der Formel

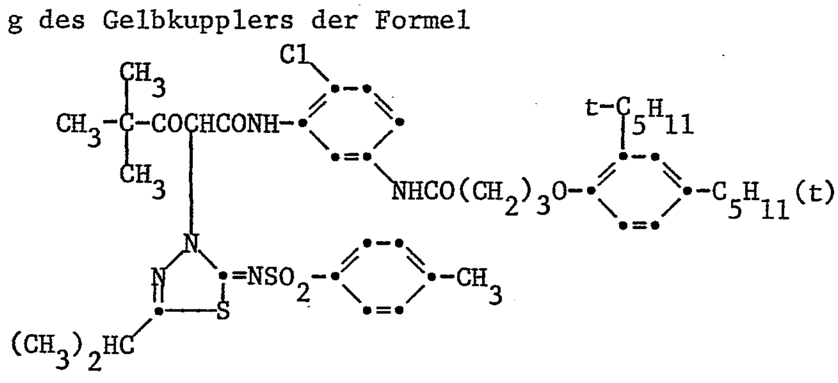

und 0,026 g eines der in den nachfolgenden Tabellen angegebenen Lichtschutzmittels werden in 2,0 ml eines Gemisches von Trikresylphosphat/Aethylacetat (1,5 g in 100 ml) gelöst. Zu dieser Lösung gibt man 7,0 ml einer 6%igen Gelatinelösung, 0,5 ml einer 8%igen Lösung des Netzmittels der Formel

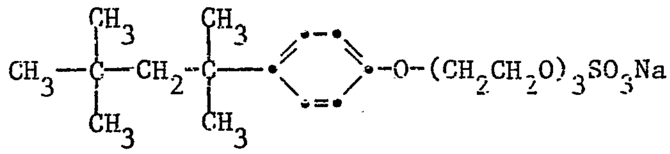

in Isopropanol/Wasser (3:4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %igen wässerigen Lösung des Härters der Formel

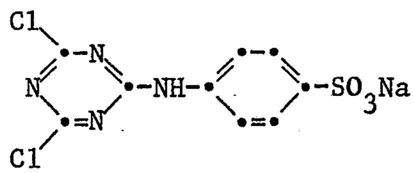

und 3,8 ml Wasser, stellt das Gemisch auf einen pH-Wert von 6,5 und vergiesst es auf ein auf eine Glasplatte aufgezogenes substituiertes, kunststoffbeschichtetes, weisses Papier.

Nach dem Erstarren wird in einem Trockenschrank mit Umluft bei Raumtemperatur getrocknet.

Nach 7 Tagen werden auf 35 x 180 mm geschnittene Proben hinter einem Stufenkeit mit 3000 Lux•s belichtet und anschliessend im Ektaprint 2®-Prozess der Firma Kodak verarbeitet.

Die so erhaltenen Gelbkeile werden in einem Atlas Weather-Ometer mit einer 2500 W-Xenonlampe mit total 42 $kJoule/cm^2$ bestrahlt (eine Vergleichsprobe enthält kein Lichtschutzmittel).

In Tabelle 1 sind die prozentualen Farbdichteabnahmen bei einer ursprünglichen Dichte von 1,0 enthalten.

Tabelle 1

| Lichtschutzmittel Nr. | Dichteverlust am Maximum in Prozent (Remission) |
|---|---|
| ohne | 41 |
| 2 | 20 |
| 3 | 16 |
| 4 | 18 |
| 5 | 19 |
| 6 | 17 |
| 7 | 19 |
| 8 | 19 |
| 9 | 18 |
| 14 | 20 |

Beispiel B

Es werden Proben wie in Beispiel 1 hergestellt, jedoch werden jeweils 0,042 mMol des Lichtschutzmittels zusammen mit dem Kuppler eingegossen.

Die Proben werden anschliessend in einen Atlas Weather-Ometer hinter einem UV-Filter (Kodak, Typ 2C) mit total 63 $KJoule/cm^2$ bestrahlt. Tabelle 2 enthält die hierbei enthaltenen prozentualen Dichteabnahmen am Maximum in Remission.

Tabelle 2

| Lichtschutzmittel Nr. | Dichteverlust am Maximum in Remission in Prozent |
|---|---|
| ohne | 15 |
| 5 | 8 |
| 9 | 6 |

Aus den Beispielen A und B ist ersichtlich, dass die Lichtschutzmittel der vorliegenden Erfindung die Lichtbeständigkeit eines chromogenen Gelbfarbstoffes deutlich verbessern.

Beispiel C: 0,025 g des Cyankupplers der Formel

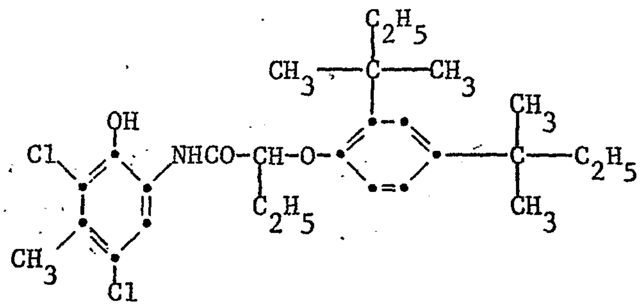

und 0,04 mMol eines Lichtschutzmittels der nachfolgenden Tabelle werden in 1 ml eines Gemisches von Trikresylphosphat-Aethylacetat (1,5 g in 100 ml) gelöst. Zu dieser Lösung gibt man 7,0 ml einer 6 %igen Gelatinelösung, 0,5 ml einer 8 %igen Lösung des Netzmittels der Formel

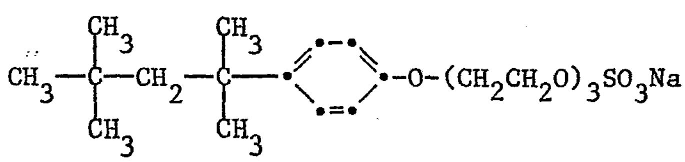

in Isopropanol/Wasser (3:4) und 0,5 ml Wasser und emulgiert mit Ultraschall bei einer Leistung von 100 Watt während 5 Minuten.

Zu 2,5 ml der so erhaltenen Emulsion gibt man 2,0 ml einer Silberbromid-Emulsion mit einem Silbergehalt von 6,0 g pro Liter, 0,7 ml einer 1 %igen wässerigen Lösung des Härters der Formel

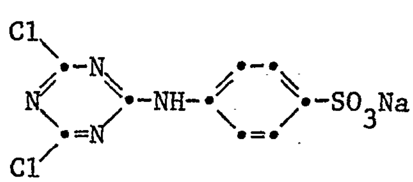

und 3,8 ml Wasser, stellt das Gemisch auf einen pH-Wert von 6,5 und vergiesst es auf ein auf eine Glasplatte aufgezogenes substituiertes, kunststoffbeschichtetes, weisses Papier.

Nach dem Erstarren wird in einem Trockenschrank mit Umluft bei Raumtemperatur getrocknet.

In Analogie zu dem in Beispiel A und B beschriebenen Verfahren werden Schichten hergestellt, belichtet und verarbeitet.

Die erhaltenen Keile werden in einem Klimaschrank bei 60°C und 70 % rel. Feuchtigkeit gelagert. Die nachfolgende Tabelle drückt die prozentuale Abnahme der Cyandichte bei einer ursprünglichen Farbdichte von 1,0 im Rot aus (Messung mit einem Densitometer® TR 924 Status A, der Firma Macbeth).

Tabelle 3

| Lichtschutzmittel Nr. | Dichteverlust im Rot in Prozent (D = 1,0, 60°C/70 % RF) | |
|---|---|---|
| | 14 Tage | 28 Tage |
| ohne | 24 | 42 |
| 2 | 14 | 23 |
| 17 | 13 | 23 |
| 18 | 9 | 19 |

Man ersieht daraus, dass die Stabilisatoren der vorliegenden Anmeldung die Klimabeständigkeit eines chromogenen Cyanfarbstoffes stark verbessern.

0103540



Patentansprüche

1. Farbphotographisches Aufzeichnungsmaterial, das in mindestens einer lichtempfindlichen Silberhalogenid-Emulsionsschicht, einer Zwischenschicht und/oder einer Schutzschicht mindestens eine Polyalkylpiperidinverbindung als Lichtschutzmittel enthält, dadurch gekennzeichnet, dass es als Lichtschutzmittel eine Verbindung der Formel I enthält

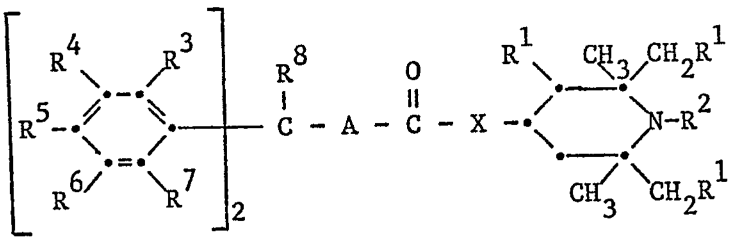 (I),

worin

$R^1$ Wasserstoff oder Methyl ist,

$R^2$ Hydroxyl, $C_1$-$C_{12}$-Alkyl, $C_3$-$C_6$-Alkenyl, $C_3$-$C_4$-Alkinyl, $C_7$-$C_{12}$-Aralkyl, Glycidyl, durch Halogen, Cyan, $-COOR^9$ oder $-CON(R^{10})(R^{11})$ substituiertes $C_1$-$C_4$-Alkyl, eine Gruppe $-CO-R^{12}$, $-CO-OR^9$ oder $-CO-N(R^{10})(R^{11})$, eine Gruppe $-CH_2-CH(R^{13})-OR^{14}$, $-SO-R^{15}$, $-SO_2-R^{15}$, $-OR^9$, $-OOC-R^{12}$ oder eine Gruppe der Formel

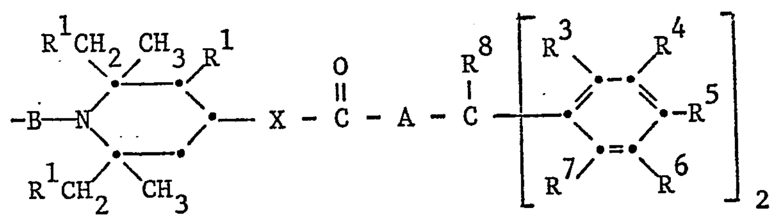

bedeutet,

$R^3$ und $R^5$ Wasserstoff oder Hydroxyl sind, wobei entweder $R^3$ Wasserstoff und $R^5$ Hydroxyl oder $R^3$ Hydroxyl und $R^5$ Wasserszoff sind,

$R^4$ $C_1$-$C_{12}$-Alkyl, $C_5$-$C_8$-Cycloalkyl, $C_6$-$C_{10}$-Aryl oder $C_7$-$C_9$-Phenylalkyl ist,

$R^6$ und $R^7$ unabhängig voneinander Wasserstoff, $C_1$-$C_{12}$-Alkyl, $C_5$-$C_8$-Cycloalkyl, $C_6$-$C_{10}$-Aryl oder $C_7$-$C_9$-Phenylalkyl bedeuten,

$R^8$ Wasserstoff, $C_1$-$C_{12}$-Alkyl, $C_5$-$C_8$-Cycloalkyl, Benzyl oder Phenyl ist,

$R^9$ $C_1$-$C_{12}$-Alkyl, Allyl, Cyclohexyl oder Benzyl bedeutet,

$R^{10}$ $C_1$-$C_{12}$-Alkyl, Allyl, Cyclohexyl, Benzyl, Phenyl oder $C_7$-$C_{12}$-Alkylphenyl bedeutet,

$R^{11}$ Wasserstoff, $C_1$-$C_{12}$-Alkyl, Allyl, Cyclohexyl oder Benzyl bedeutet oder

$R^{10}$ und $R^{11}$ zusammen mit dem N-Atom, an das sie gebunden sind, einen 5- oder 6-gliedrigen heterocyclischen Ring bilden,

$R^{12}$ Wasserstoff, $C_1$-$C_{12}$-Alkyl, $C_2$-$C_6$-Alkenyl, Chlormethyl, $C_5$-$C_8$-Cycloalkyl, $C_7$-$C_{12}$-Aralkyl, Phenyl, $C_7$-$C_{12}$-Alkylphenyl oder durch 1 oder 2 $C_1$-$C_4$-Alkylgruppen und eine Hydroxygruppe substituiertes Phenyl, Phenylmethyl oder Phenylethyl bedeutet,

$R^{13}$ Wasserstoff, $C_1$-$C_4$-Alkyl, $C_2$-$C_{13}$-Alkoxymethyl, Phenyl oder Phenoxymethyl bedeutet,

$R^{14}$ Wasserstoff, $C_1$-$C_{12}$-Alkyl, eine Gruppe $-CO-R^{12}$, $-CO-N(R^{10})(R^{11})$ oder eine Gruppe der Formel II bedeutet

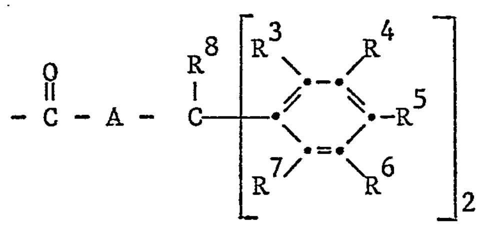

(II),

$R^{15}$ $C_1$-$C_{12}$-Alkyl, Phenyl oder $C_7$-$C_{12}$-Alkylphenyl ist,

A eine direkte Bindung oder $C_1$-$C_{12}$-Alkylen, das durch -S- oder -COOunterbrochen oder durch eine der Gruppen $-SO_2-R^{15}$, -CN, $-P(O)(OR^{15})_2$ oder

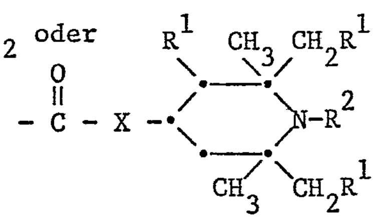

substituiert sein kann, bedeutet,

B $C_2$-$C_{12}$-Alkylen, $C_4$-$C_8$-Alkenylen, $C_4$-$C_6$-Alkinylen oder $C_8$-$C_{14}$-Aralkylen bedeutet, und

X -O- oder $-N(R^{16})-$ bedeutet, worin

$R^{16}$ Wasserstoff, $C_1$-$C_{18}$-Alkyl, $C_5$-$C_{12}$-Cycloalkyl oder eine Gruppe der Formel III

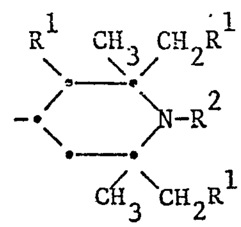

(III)

bedeutet.

2. Farbphotographisches Aufzeichnungsmaterial gemäss Anspruch 1, dadurch gekennzeichnet, dass das Lichtschutzmittel eine Verbindung der Formel I ist, worin $R^1$ Wasserstoff ist, $R^2$ Hydroxy, $C_1$-$C_4$-Alkoxy, Acetoxy, $C_1$-$C_4$-Alkyl, $C_3$-$C_4$-Alkenyl, Propargyl, Glycidyl, Benzyl, durch $-COOR^9$ substituiertes Methyl oder Ethyl, eine Gruppe $-CO-R^{12}$, $-CO-OR^9$ oder $-CO-N(R^{10})(R^{11})$, eine Gruppe $-CH_2-CH(R^{13})-OR^{14}$, $-SO-R^{15}$, $-SO_2-R^{15}$, $-OR^9$ oder $-OOC-R^{12}$ bedeutet, $R^3$ Wasserstoff und $R^5$ Hydroxyl oder $R^3$ Hydroxyl und $R^5$ Wasserstoff sind, $R^4$ $C_1$-$C_8$-Alkyl ist, $R^6$ und $R^7$ unabhängig voneinander H oder $C_1$-$C_8$-Alkyl sind, $R^8$ Wasserstoff oder $C_1$-$C_{12}$-Alkyl ist, $R^9$ $C_1$-$C_8$-Alkyl, Allyl oder Cyclohexyl ist, $R^{10}$ $C_1$-$C_{12}$-Alkyl, Cyclohexyl oder Phenyl ist, $R^{11}$ Wasserstoff oder $C_1$-$C_{12}$-Alkyl ist oder $R^{10}$ und $R^{11}$ zusammen mit dem N-Atom, an das sie gebunden sind, einen 6-gliedrigen heterocyclischen Ring bilden, $R^{12}$ $C_1$-$C_{12}$-Alkyl, $C_2$-$C_4$-Alkenyl, Cyclohexyl, Benzyl, Phenyl oder 2-(3,5-Di-tert.butyl-4-hydroxyl)-ethyl ist, $R^{13}$ Wasserstoff, Methyl oder Phenyl ist, $R^{14}$ Wasserstoff, $C_1$-$C_4$-Alkyl, eine Gruppe $-CO-R^{12}$, $-CO-N(R^{10})(R^{11})$ oder eine Gruppe der Formel II ist, $R^{15}$ $C_1$-$C_4$-Alkyl, Phenyl oder Tolyl ist, A eine direkte Bindung oder $C_1$-$C_6$-Alkylen, das durch -CN oder eine Gruppe der Formel IV

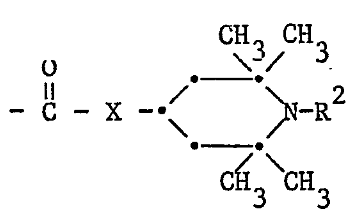

(IV)

substituiert sein kann und X -O- oder -NH- bedeutet.

3. Farbphotographisches Aufzeichnungsmaterial gemäss Anspruch 1, dadurch gekennzeichnet, dass das Lichtschutzmittel eine Verbindung der Formel I ist, worin $R^1$ Wasserstoff ist, $R^2$ Methoxy, Methyl, Allyl, Benzyl, Acetyl, Acryloyl, eine Gruppe $-CO-N(R^{10})(R^{11})$, $-SO-R^{15}$ oder $-SO_2-R^{15}$ ist, $R^3$ Wasserstoff ist, $R^4$ $C_1$-$C_6$-Alkyl, $R^5$ Hydroxyl, $R^6$ und $R^7$ unabhängig voneinander Wasserstoff oder $C_1$-$C_4$-Alkyl sind, $R^8$ Wasserstoff oder $C_1$-$C_6$-Alkyl ist, $R^{10}$ $C_1$-$C_8$-Alkyl, Cyclohexyl oder Phenyl und $R^{11}$ Wasserstoff oder $C_1$-$C_8$-Alkyl ist, $R^{15}$ Methyl, Phenyl oder p-Tolyl ist, A eine direkte Bindung oder $C_1$-$C_4$-Alkylen ist, das durch eine Gruppe der Formel IV substituiert sein kann, und X -O- oder -NH- bedeutet.

4. Farbphotographisches Aufzeichnungsmaterial gemäss Anspruch 1, dadurch gekennzeichnet, dass das Lichtschutzmittel eine Verbindung der Formel I ist, worin $R^1$ Wasserstoff ist, $R^2$ Methoxy, Methyl, Allyl, Benzyl, Acetyl, Acryloyl, oder eine Gruppe $-CO-N(R^{10})(R^{11})$, $-SO-R^{15}$ oder $-SO_2-R^{15}$ ist, $R^3$ Wasserstoff, $R^4$ $C_1$-$C_4$-Alkyl und $R^5$ Hydroxyl sind, $R^6$ Wasserstoff oder $C_1$-$C_4$-Alkyl ist, $R^7$ und $R^8$ Wasserstoff oder Methyl sind, $R^{10}$ $C_1$-$C_4$-Alkyl, Cyclohexyl oder Phenyl ist, $R^{11}$ Wasserstoff oder $C_1$-$C_4$-Alkyl ist, $R^{15}$ Phenyl oder p-Tolyl ist, A eine direkte Bindung oder $C_1$-$C_4$-Alkylen ist und X -O- ist.

5. Farbphotographisches Aufzeichnungsmaterial gemäss Anspruch 1, dadurch gekennzeichnet, dass das Lichtschutzmittel eine Verbindung der Formel I ist, worin $R^1$ Wasserstoff ist, $R^2$ Methyl, Allyl, Benzyl, Acetyl oder Acryloyl ist, $R^3$ Wasserstoff, $R^4$ $C_1$-$C_4$-Alkyl, $R^5$ Hydroxyl und $R^6$, $R^7$ und $R^8$ Wasserstoff oder Methyl sind, A $C_1$-$C_4$-Alkylen ist und X -O- ist.

6. Farbphotographisches Aufzeichnungsmaterial gemäss Anspruch 1, dadurch gekennzeichnet, dass dieses ausser einem Lichtschutzmittel der Formel I noch ein oder mehrere andere Lichtschutzmittel enthält.

7. Farbphotographisches Aufzeichnungsmaterial gemäss Anspruch 6, dadurch gekennzeichnet, dass dieses ausser dem Lichtschutzmittel der Formel I noch einen Ultraviolett-Absorber enthält.

8. Farbphotographisches Aufzeichnungsmaterial gemäss Anspruch 1, dadurch gekennzeichnet, dass es das oder die Lichtschutzmittel in Kombination mit einem oder mehreren Farbkupplern enthält.

9. Farbphotographisches Aufzeichnungsmaterial gemäss Anspruch 1, dadurch gekennzeichnet, dass es pro $m^2$ 1 bis 2000, vorzugsweise 100 bis 800 mg Lichtschutzmittel enthält.

FO 7.3 SA/cw*

Europäisches Patentamt

# EUROPÄISCHER RECHERCHENBERICHT

Nummer der Anmeldung

EP 83 81 0357

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A,D | FR-A-2 349 574 (CIBA-GEIGY)<br>--- | | G 03 C 7/26 //<br>C 07 D 211/46<br>C 07 D 211/58 |
| A,D | EP-A-0 011 051 (CIBA-GEIGY)<br>----- | | |
| | | | RECHERCHIERTE SACHGEBIETE (Int. Cl. 3) |
| | | | G 03 C 7/00<br>G 03 C 1/00 |

Der vorliegende Recherchenbericht wurde für alle Patentansprüche erstellt.

| Recherchenort | Abschlußdatum der Recherche | Prüfer |
|---|---|---|
| DEN HAAG | 15-12-1983 | PHILOSOPH L.P. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument